# FASCICULE DE BREVET EUROPEEN

(11) **EP 0 994 948 B1**
(45) Date de publication et mention de la délivrance du brevet: **16.05.2001**
(21) Numéro de dépôt: 99923698.7
(22) Date de dépôt: 08.06.1999
(51) Int. Cl.: C12N 15/12, C07K 14/71, C12N 15/70, C12N 1/21, A61K 48/00, A61K 38/17

(54) **SEQUENCE NUCLEIQUE DU RECEPTEUR RII DU TGFSS, PEPTIDE CODE, ET UTILISATIONS**
TGF-BETA RII REZEPTOR NUKLEINSÄURESEQUENZ, DADURCH KODIERTES PEPTID, UND VERWENDUNGEN DAVON
TGF BETA RII RECEPTOR NUCLEIC SEQUENCE, CODED PEPTIDE, AND USES

(30) Priorité: 08.06.1998 FR 9807322
(43) Date de publication de la demande: 26.04.2000
(73) Titulaire: TRANSGENE S.A., 67000 Strasbourg (FR)
(72) Inventeur: ACRES, Bruce, F-67000 Strasbourg (FR)
(74) Mandataire: Guerre, Dominique
(86) Numéro de dépôt international: FR9901354
(87) Numéro de publication internationale: WO9964588

(56) Documents cités:
- WO-A-96/31605

### LISTAGE DE SEQUENCES

(1) INFORMATIONS GENERALES:
   (i) DEPOSANT:
      (A) NOM: TRANSGENE
      (B) RUE: 11, rue de Molsheim
      (C) VILLE: STRASBOURG
      (E) PAYS: FRANCE
      (F) CODE POSTAL: 67082 cedex
   (ii) TITRE DE L'INVENTION:
   (iii) NOMBRE DE SEQUENCES: 43
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patent In Release #1.0, Version #1.30 (OEB)
(2) INFORMATIONS POUR LA SEQ ID NO:1 :
   (i) CARACTERISTIQUES DE LA SEQUENCE:
      (A) LONGUEUR: 34 résidus d'acide aminé
      (B) TYPE: acides aminés
   (ii) TYPE DE MOLECULE: peptide
   (xi) DESCRIPTION DE LA SEQUENCE: SEQ ID NO:1 :
   Ser Leu Val Arg Leu Ser Ser Cys Val Pro Val Ala Leu Met Ser Ala
   1               5                   10                  15
   Met Thr Thr Ser Ser Ser Gln Lys Asn Ile Thr Pro Ala Ile Leu Thr
               20                  25                  30
   Cys Cys
(2) INFORMATIONS POUR LA SEQ ID NO:2 :
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO:2 :
   Ser Leu Val Arg Leu Ser Ser Cys Val
   1               5
(2) INFORMATIONS POUR LA SEQ ID NO: 3:
   (i) CARACTERISTIQUES DE LA SEQUENCE:
      (A) LONGUEUR: 11 résidus d'acide aminé
      (B) TYPE: acides aminés
   (ii) TYPE DE MOLECULE: peptide
   (xi) DESCRIPTION DE LA SEQUENCE: SEQ ID NO: 3:
   Ser Leu Val Arg Leu Ser Ser Cys Val Pro Val
   1               5                   10
(2) INFORMATIONS POUR LA SEQ ID NO: 4:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 4:
   Arg Leu Ser Ser Cys Val Pro Val
   1               5
(2) INFORMATIONS POUR LA SEQ ID NO: 5:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 5:
   Arg Leu Ser Ser Cys Val Pro Val Ala
   1               5
(2) INFORMATIONS POUR LA SEQ ID NO: 6:
   (i) CARACTERISTIQUES DE LA SEQUENCE:
      (A) LONGUEUR: 10 résidus d'acide aminé
      (B) TYPE: acides aminés
   (ii) TYPE DE MOLECULE: peptide
   (xi) DESCRIPTION DE LA SEQUENCE: SEQ ID NO: 6:
   Arg Leu Ser Ser Cys Val Pro Val Ala Leu
   1               5
(2) INFORMATIONS POUR LA SEQ ID NO: 7:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 7:
   Met Thr Thr Ser Ser Ser Gln Lys
   1               5
(2) INFORMATIONS POUR LA SEQ ID NO: 8:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 8:
   Ala Met Thr Thr Ser Ser Ser Gln Lys
   1               5
(2) INFORMATIONS POUR LA SEQ ID NO: 9:
   (i) CARACTERISTIQUES DE LA SEQUENCE:
      (A) LONGUEUR: 10 résidus d'acide aminé
      (B) TYPE: acides aminés
   (ii) TYPE DE MOLECULE: peptide
   (xi) DESCRIPTION DE LA SEQUENCE: SEQ ID NO: 9:
   Ser Ala Met Thr Thr Ser Ser Ser Gln Lys
   1               5                   10
(2) INFORMATIONS POUR LA SEQ ID NO: 10:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 10:
   Ile Met Lys Glu Lys Lys Ser Leu
   1               5
(2) INFORMATIONS POUR LA SEQ ID NO: 11:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 11:
   Ile Met Lys Glu Lys Lys Ser Leu Val
   1               5
(2) INFORMATIONS POUR LA SEQ ID NO: 12:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 12:
   Lys Ser Leu Val Arg Leu Ser Ser
   1               5
(2) INFORMATIONS POUR LA SEQ ID NO: 13:
   (i) CARACTERISTIQUES DE LA SEQUENCE:
      (A) LONGUEUR: 10 résidus d'acide aminé
      (B) TYPE: acides aminés
   (ii) TYPE DE MOLECULE: peptide
   (xi) DESCRIPTION DE LA SEQUENCE: SEQ ID NO: 13:
   Lys Ser Leu Val Arg Leu Ser Ser Cys Val
   1               5                   10
(2) INFORMATIONS POUR LA SEQ ID NO: 14:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 14:
   Thr Ser Ser Ser Gln Lys Asn Ile
   1               5
(2) INFORMATIONS POUR LA SEQ ID NO: 15:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 15:
   Ser Ser Ser Gln Lys Asn Ile Thr
   1               5
(2) INFORMATIONS POUR LA SEQ ID NO: 16:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 16:
   Thr Ser Ser Ser Gln Lys Asn Ile Thr
   1               5
(2) INFORMATIONS POUR LA SEQ ID NO: 17:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 17:
   Cys Ile Met Lys Glu Lys Lys Ser Leu
   1               5
(2) INFORMATIONS POUR LA SEQ ID NO: 18:
   (i) CARACTERISTIQUES DE LA SEQUENCE:
      (A) LONGUEUR: 8 résidus d'acide aminé
      (B) TYPE: acides aminés
   (ii) TYPE DE MOLECULE: peptide
   (xi) DESCRIPTION DE LA SEQUENCE: SEQ ID NO: 18:
   Val Pro Val Ala Leu Met Ser Ala
   1               5
(2) INFORMATIONS POUR LA SEQ ID NO: 19:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 19:
   Leu Ser Ser Cys Val Pro Val Ala Leu
   1               5
(2) INFORMATIONS POUR LA SEQ ID NO: 20:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 20:
   Val Pro Val Ala Leu Met Ser Ala Met
   1 5
(2) INFORMATIONS POUR LA SEQ ID NO: 21:
   (i) CARACTERISTIQUES DE LA SEQUENCE:
      (A) LONGUEUR: 9 résidus d'acide aminé
      (B) TYPE: acides aminés
   (ii) TYPE DE MOLECULE: peptide
   (xi) DESCRIPTION DE LA SEQUENCE: SEQ ID NO: 21:
   Val Arg Leu Ser Ser Cys Val Pro Val
   1 5
(2) INFORMATIONS POUR LA SEQ ID NO: 22
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 22:
   AGCCTGGTGA GACTTTCTTC ATGTGTT 27
(2) INFORMATIONS POUR LA SEQ ID NO: 23
   (i) CARACTERISTIQUES DE LA SEQUENCE:
      (A) LONGUEUR: 33 paires de bases
      (B) TYPE: nucléotide
   (ii) TYPE DE MOLECULE: ADNc
   (xi) DESCRIPTION DE LA SEQUENCE: SEQ ID NO: 23:
   AGCCTGGTGA GACTTTCTTC ATGTGTTCCT GTA 33
(2) INFORMATIONS POUR LA SEQ ID NO: 24
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 24:
   AGACTTTCTT CATGTGTTCC TGTA 24
(2) INFORMATIONS POUR LA SEQ ID NO: 25
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 25:
   AGACTTTCTT CATGTGTTCC TGTAGCT 27
(2) INFORMATIONS POUR LA SEQ ID NO: 26
   (i) CARACTERISTIQUES DE LA SEQUENCE:
      (A) LONGUEUR: 30 paires de bases
      (B) TYPE: nucléotide
   (ii) TYPE DE MOLECULE: ADNc
   (xi) DESCRIPTION DE LA SEQUENCE: SEQ ID NO: 26:
   AGACTTTCTT CATGTGTTCC TGTAGCTCTG 30
(2) INFORMATIONS POUR LA SEQ ID NO: 27
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 27:
   ATGACAACAT CATCTTCTCA GAAG 24
(2) INFORMATIONS POUR LA SEQ ID NO: 28
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 28:
   GCAATGACAA CATCATCTTC TCAGAAG 27
(2) INFORMATIONS POUR LA SEQ ID NO: 29
   (i) CARACTERISTIQUES DE LA SEQUENCE:
      (A) LONGUEUR: 30 paires de bases
      (B) TYPE: nucléotide
   (ii) TYPE DE MOLECULE: ADNc
   (xi) DESCRIPTION DE LA SEQUENCE: SEQ ID NO: 29:
   AGTGCAATGA CAACATCATC TTCTCAGAAG 30
(2) INFORMATIONS POUR LA SEQ ID NO: 30
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 30:
   ATTATGAAGG AAAAAAAAAG CCTG 24
(2) INFORMATIONS POUR LA SEQ ID NO: 31
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 31:
   ATTATGAAGG AAAAAAAAAG CCTGGTG 27
(2) INFORMATIONS POUR LA SEQ ID NO: 32
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 32:
   AAAAGCCTGG TGAGACTTTC TTCA 24
(2) INFORMATIONS POUR LA SEQ ID NO: 33
   (i) CARACTERISTIQUES DE LA SEQUENCE:
      (A) LONGUEUR: 30 paires de bases
      (B) TYPE: nucléotide
   (ii) TYPE DE MOLECULE: ADNc
   (xi) DESCRIPTION DE LA SEQUENCE: SEQ ID NO: 33:
   AAAAGCCTGG TGAGACTTTC TTCATGTGTT 30
(2) INFORMATIONS POUR LA SEQ ID NO: 34
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 34:
   ACATCATCTT CTCAGAAGAA TATA 24
(2) INFORMATIONS POUR LA SEQ ID NO: 35
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 35
   TCATCTTCTC AGAAGAATAT AACA 24
(2) INFORMATIONS POUR LA SEQ ID NO: 36
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 36
   ACATCATCTT CTCAGAAGAA TATAACA 27
(2) INFORMATIONS POUR LA SEQ ID NO: 37
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 37
   TGCATTATGA AGGAAAAAAA AAGCCTG 27
(2) INFORMATIONS POUR LA SEQ ID NO: 38
   (i) CARACTERISTIQUES DE LA SEQUENCE:
      (A) LONGUEUR: 24 paires de bases
      (B) TYPE: nucléotide
   (ii) TYPE DE MOLECULE: ADNc
   (xi) DESCRIPTION DE LA SEQUENCE: SEQ ID NO: 38
   GTTCCTGTAG CTCTGATGAG TGCA 24
(2) INFORMATIONS POUR LA SEQ ID NO: 39
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 39
   CTTTCTTCAT GTGTTCCTGT AGCTCTG 27
(2) INFORMATIONS POUR LA SEQ ID NO: 40
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 40
   GTTCCTGTAG CTCTGATGAG TGCAATG 27
(2) INFORMATIONS POUR LA SEQ ID NO: 41
   (i) CARACTERISTIQUES DE LA SEQUENCE:
      (A) LONGUEUR: 27 paires de bases
      (B) TYPE: nucléotide
   (ii) TYPE DE MOLECULE: ADNc
   (xi) DESCRIPTION DE LA SEQUENCE: SEQ ID NO: 41
   GTGAGACTTT CTTCATGTGT TCCTGTA                                          27
(2) INFORMATIONS POUR LA SEQ ID NO: 42
   (i) CARACTERISTIQUES DE LA SEQUENCE:
      (A) LONGUEUR: 123 paires de bases
      (B) TYPE: nucléotide
   (ii) TYPE DE MOLECULE: ADNc
   (xi) DESCRIPTION DE LA SEQUENCE: SEQ ID NO: 42
   TGCATTATGA AGGAAAAAAA AAGCCTGGTG AGACTTTCTT CATGTGTTCC TGTAGCTCTG   60
   ATGAGTGCAA TGACAACATC ATCTTCTCAG AAGAATATAA CACCAGCAAT CCTGACTTGT  120
   TGC                                                                123
(2) INFORMATIONS POUR LA SEQ ID NO: 43
   (i) CARACTERISTIQUES DE LA SEQUENCE:
      (A) LONGUEUR: 26 résidus d'acide aminé
      (B) TYPE: acides aminés
   (ii) TYPE DE MOLECULE: peptide
   (xi) DESCRIPTION DE LA SEQUENCE: SEQ ID NO: 43:
   Tyr Lys Lys Pro Val Ala Leu Met Ser Ala Met Thr Thr Ser Ser Ser
   1               5                   10                  15
   Gln Lys Asn Ile Thr Pro Ala Ile Leu Thr
               20                  25

## Description

La réponse immune dirigée contre un antigène spécifique peut être divisée en deux catégories distinctes, l'une mettant en jeu les anticorps (réponse immune de type humoral), l'autre les lymphocytes T (réponse immune de type cellulaire). Plus particulièrement, les deux types de réponses se distinguent en ce que les anticorps reconnaissent les antigènes sous leur forme tridimensionnelle, alors que les lymphocytes T reconnaissent des portions peptidiques desdits antigènes, associés à des glycoprotéines codées par les gènes du complexe majeur d'histocompatibilité (ou CMH), notamment les gènes du complexe majeur d'histocompatibilité de type I, qui sont exprimés de façon ubiquitaire à la surface des cellules, ou les gènes du complexe majeur d'histocompatibilité de type II, qui sont exprimés de façon spécifique à la surface des cellules impliquées dans la présentation des antigènes (APC).

Selon un premier aspect, la réponse immune de type cellulaire est caractérisée en ce que les cellules T de type CD4⁺ (cellules T « helper »), après activation par les fragments antigéniques présentés par les APC, produisent des cytokines qui à leur tour induisent la prolifération de cellules APC capables de produire lesdites cytokines ; la différenciation cellulaire des lymphocytes B capables de produire des anticorps spécifiques ; prolifération des cellules T CD4 et la stimulation des lymphocytes cytotoxiques (CTL).

Selon un second aspect de la réponse immune cellulaire, les lymphocytes cytotoxiques de type CD8⁺ (CTL) sont activés a) après interaction avec des peptides antigéniques fixés sur et présentés par les glycoprotéines portées par les cellules ubiquitaires et codées par les gènes appartenant au système CMH I des APC avec des molécules co-stimulatrices, et b) éventuellement par les cytokines produites par les CD4⁺. Les CTL ainsi activés sont alors capables de détruire les cellules exprimant ledit peptide antigénique associé avec les glycoprotéines du CMH I.

La présentation des fragments antigéniques par les molécules CMH I repose sur un procédé intracellulaire identifié (voir Groettrup et al., 1996, Immunology Today, 17, 429-435 pour une revue) au cours duquel des peptides antigéniques de très courtes tailles (environ 7-13 acides aminés) sont produits par dégradation d'un polypeptide plus complexe contre lequel la réaction immune finale sera dirigée (par exemple une protéine virale). Ces courts peptides sont ensuite associés aux molécules CMH I pour former un complexe trimoléculaire qui est transporté à la surface cellulaire afin de présenter lesdits peptides aux lymphocytes T cytotoxiques circulants. Il convient en outre de noter que la spécificité des molécules CMH I vis-à-vis des peptides antigéniques varie en fonction de la molécule CMH I (HLA-A, HLA-B,...) et de l'allèle (HLA-A2, HLA-A3, HLA-A11,...) considérés. Au sein d'une même espèce animale, d'un individu à l'autre, il existe une grande variabilité des gènes codant pour les molécules du système CMH (à ce sujet, voir notamment George et al., 1995, Immunology Today, 16, 209-212).

Dans le cas des cancers, Hellstrom et al., (1969, Adv. Cancer Res. 12, 167-223) ont montré que la défense de l'organisme à l'égard des tumeurs repose tout particulièrement sur la réponse immunitaire mettant en jeu les lymphocytes T, notamment les lymphocytes T cytotoxiques. Toutefois, il apparait que cette réaction immune naturelle n'est pas suffisamment efficace pour empêcher le développement tumoral. C'est la raison pour laquelle il est souhaitable de disposer d'une méthode d'activation de la réponse immune dirigée contre les tumeurs, et plus particulièrement de la réponse faisant intervenir les lymphocytes cytotoxiques CTL, afin de disposer de méthodes de prévention ou de traitement des états cancéreux plus efficaces.

Les cellules tumorales expriment des antigènes spécifiques qui ne sont pas présents ou en quantité moindre dans les cellules normales, et de nombreux travaux ont montré que ces antigènes peuvent être les cibles de réactions anti-tumorales observées par exemple après immunisation de l'organisme avec la protéine antigénique totale, avec des fragments peptidiques d'une telle protéine, après administration d'ADN codant pour tout ou partie de ladite protéine, en particulier après administration de vecteurs viraux recombinants capables d'exprimer un tel antigène spécifique de tumeurs.

Toutefois, cette approche vaccinale anti-tumorale n'est pas toujours satisfaisante et conduit par exemple à des réactions immunes limitées dirigées uniquement à l'encontre d'épitopes immunodominants ou contre des antigènes présentant une grande variabilité. De même, la présentation incorrecte des antigènes par les glycoprotéines du système CMH à la surface des cellules, ne permet pas de développer chez le patient traité une immunité anti-tumorale convenable.

Afin de pallier à ces problèmes, certains auteurs ont proposé, dans le cadre de tels procédés vaccinaux, de sélectionner les fragments minimaux antigéniques correspondant aux portions de peptide susceptibles d'être reconnus spécifiquement par les lymphocytes T cytotoxiques, de les exprimer dans les cellules afin qu'ils s'associent aux molécules du CMH I et soient présentés à la surface des cellules pour induire chez le patient traité une réaction immunitaire parfaitement ciblée (Toes et al., 1997, Proc. Natl. Acad. Sci., 94, 14660-14665). Plus particulièrement, il a été montré que des épitopes de très petites tailles (variant de 7 à environ 13 acides aminés) qui sont exprimés à partir de minigènes introduits dans un virus de la vaccine, pouvaient induire une immunisation de type cellulaire. Il a par ailleurs été montré que plusieurs minigènes pouvaient être exprimés conjointement à partir d'un même vecteur (cette construction particulière est appelée « string of beads »). Une telle construction présente l'avantage d'induire une réaction immune de type CTL synergique (Whitton et al, 1993, J. Of Virology, 67, 348-352).

La présente invention concerne des séquences nucléiques identifiées à partir de la séquence nucléique codant pour le mutant du récepteur RII du facteur de croissance transformant (TGFβ) (WO-A-96/31605), les vecteurs permettant le transfert et l'expression de ces séquences nucléiques à l'intérieur des cellules cibles, les peptides immunoréactifs exprimés par ces séquences, ainsi que les utilisations desdites séquences nucléiques et dits peptides.

Les cancers du colon et les cancers gastriques font partie des cancers les plus fréquents. On sait qu'une forte proportion de ces cancers est liée à un dysfonctionnement du facteur TGFβ qui perd sa fonction normale d'inhibition de la croissance de différents types cellulaires.

On a en fait observé, dans ces cas, différentes séquences nucléiques du récepteur RII du TGFβ qui présentent, par rapport à la séquence de référence, un décalage du cadre de lecture. L'expression de certaines de ces séquence nucléiques « décalées » codant pour des protéines différentes de la protéine naturellement codée par la séquence nucléique de référence (dénommées formes mutantes du récepteur RII du TGFβ) entraîne une inactivation du TGFβ qui contribuerait au développement des tumeurs.

Plus particulièrement, conformément au document WO-A-96/31605, on a identifié, pour certaines tumeurs, une mutation de la séquence nucléique du récepteur RII consistant en une addition ou suppression d'au moins un nucléotide adénine dans la séquence polyadénine localisée à la position 709-718 dans la séquence de référence dudit récepteur. Cette séquence nucléique « décalée » code pour un polypeptide plus court (161 acides aminés) que le polypeptide codé par la séquence de référence, dont la séquence diffère au niveau de l'extrémité C-terminale et comporte désormais les 34 acides aminés présentés à la séquence SEQ ID NO: 1 définie dans le listage de séquences, en fin de description.

Le déposant a maintenant identifié des séquences nucléiques codant pour un peptide, ci-après défini et qui constitue un premier objet de l'invention. Ledit peptide selon l'invention comprend ou consiste en une première séquence peptidique d'au plus 20 acides aminés consécutifs définis dans une seconde séquence peptidique codée par tout ou partie de SEQ ID NO: 42 et différente de SEQ ID NO: 1, ledit peptide étant capable de se fixer sur au moins une glycoprotéine du CMH I.

Particulièrement, la première séquence définissant le peptide de l'invention est choisi parmi SEQ ID NOS: 2 à 21.

De manière avantageuse, un peptide de l'invention répond à au moins l'une quelconque des caractéristiques suivantes :
(a) la première séquence est choisie parmi SEQ ID NOS: 2 à 6, et ledit peptide est capable de se fixer sur les glycoprotéines HLA-A2 du CMH I,
(b) la première séquence est choisie parmi SEQ ID NOS: 7 à 9, et ledit peptide est capable de se fixer sur les glycoprotéines HLA-A3 du CMH I,
(c) la première séquence est choisie parmi SEQ ID NOS: 7 à 9, et ledit peptide est capable de se fixer sur les glycoprotéines HLA-A11 du CMH I,
(d) la première séquence est choisie parmi SEQ ID NOS: 10 à 17, et ledit peptide est capable de se fixer sur les glycoprotéines HLA-B8 du CMH I,
(e) la première séquence est choisie parmi SEQ ID NOS: 18 et 19, et ledit peptide est capable de se fixer sur les glycoprotéines HLA-B7 du CMH I,
(f) la première séquence est SEQ ID NO: 20, et ledit peptide est capable de se fixer sur les glycoprotéines HLA-B35 du CMH I,
(g) la première séquence est SEQ ID NO: 21, et ledit peptide est capable de se fixer sur les glycoprotéines HLA-B27 du CMH I,
(h) la première séquence est SEQ ID NO: 2, et ledit peptide est capable de se fixer sur les glycoprotéines HLA-B62 du CMH I.

Un second objet de l'invention consiste en une séquence nucléique codant pour un peptide de l'invention tel que défini ci-dessus.

Par « séquences nucléiques », on entend désigner un fragment d'ADN et/ou d'ARN, double brin ou simple brin, linéaire ou circulaire, naturel isolé ou de synthèse, désignant un enchaînement défini de nucléotides, modifiés ou non, permettant de définir une séquence susceptible de coder, en présence d'un système approprié d'expression, pour un peptide selon la présente invention.

Les séquences nucléiques préférées sont celles choisies parmi SEQ ID NO: 22 à 41 et leurs séquences complémentaires.

La présente invention concerne également des vecteurs de clonage ou d'expression comportant au moins une séquence nucléique telle que décrite précédemment.

Selon des modes de réalisations particulièrement avantageux, undit vecteur comporte au moins deux séquences nucléiques différentes respectivement choisies parmi les séquences suivantes :
les séquences codant pour un peptide tel que défini en (a), d'une part, et les séquences codant pour un peptide tel que défini en (b), (c), (d), (e), (f), (g) ou (h), d'autre part,
les séquences codant pour un peptide tel que défini en (b), d'une part, et les séquences codant pour un peptide tel que défini en (a) , (c), (d) , (e) , (f) , (g) ou (h), d'autre part,
les séquences codant pour un peptide tel que défini en (c), d'une part, et les séquences codant pour un peptide tel que défini en (a), (b), (d), (e), (f), (g) ou (h), d'autre part,
les séquences codant pour un peptide tel que défini en (d), d'une part, et les séquences codant pour un peptide tel que défini en (a), (b), (c), (e), (f), (g) ou (h), d'autre part,
les séquences codant pour un peptide tel que défini en (e), d'une part, et les séquences codant pour un peptide tel que défini en (a), (b), (c), (d), (f), (g) ou (h), d'autre part,
les séquences codant pour un peptide tel que défini en (f), d'une part, et les séquences codant pour un peptide tel que défini en (a), (b) , (c), (d), (e), (g) ou (h), d'autre part,
les séquences codant pour un peptide tel que défini en (g), d'une part, et les séquences codant pour un peptide tel que défini en (a), (b), (c), (d), (e), (f) ou (h), d'autre part, et
les séquences codant pour un peptide tel que défini en (h), d'une part, et les séquences codant pour un peptide tel que défini en (a), (b), (c), (d), (e), (f) ou (g), d'autre part.

Concernant plus particulièrement les vecteurs d'expression, ceux-ci comportent en outre des éléments assurant l'expression de ladite séquence nucléique dans une cellule hôte, notamment des séquences promotrices et/ou des séquences de régulation efficaces dans ladite cellule. A titre d'exemple, on mentionnera les promoteurs tels que les promoteurs des virus RSV, MPSV, SV40, CMV ou 7.5k, du virus de la vaccine, les promoteurs du gène codant pour la créatine kinase musculaire, pour l'actine, pour le surfactant pulmonaire. Il est en outre possible de choisir une séquence promotrice spécifique d'un type cellulaire donné, ou activable dans des conditions définies. La littérature procure un grand nombre d'informations relatives à de telles séquences promotrices.

Par ailleurs, les acides nucléiques selon la présente invention peuvent également être des acides nucléiques modifiés de sorte qu'il ne leur est pas possible de s'intégrer dans le génôme de la cellule cible ou des acides nucléiques stabilisés à l'aide d'agents, tels que par exemple la spermine.

Le vecteur en cause peut être choisi parmi les vecteurs viraux à réplication autonome, tels que les vecteurs réalisés sur la base de virus pouvant notamment être des adénovirus, des poxvirus, des virus dérivés de la vaccine, ou des virus herpétiques, ou parmi les vecteurs viraux permettant l'intégration chromosomique, tels que les rétrovirus ou les AAV (Association Adénovirus Virus), ou bien encore parmi les vecteurs non viraux consistant soit en un acide nucléique, de préférence un ADN sous forme de plasmide, éventuellement associé à au moins un composé favorisant son introduction dans les cellules tel que par exemple un lipide cationique ou un polymère cationique, à un solvant ou tout autre composé facilitant l'introduction dudit acide nucléique à l'intérieur des cellules. La littérature procure un nombre important d'exemples de tels vecteurs viraux et non viraux.

L'invention concerne également des cellules hôtes comprenant au moins une séquence nucléique ou au moins un vecteur de l'invention. Plus particulièrement, elle concerne des cellules procaryotes, des cellules de levure et des cellules animales, en particulier des cellules de mammifère, transformées par au moins un vecteur tel que décrit précédemment.

Un autre objet de l'invention est une composition pharmaceutique ou médicament, comprenant à titre de principe actif au moins une séquence nucléique, un vecteur ou une cellule hôte de l'invention. Il s'agit notamment d'une composition vaccinale, utilisable dans le cadre d'une immunothérapie par transfert de gène. L'administration à un patient d'un tel médicament permet en particulier d'induire une réponse immunitaire reposant sur l'activation des lymphocytes cytotoxiques par les peptides codés par lesdites séquences nucléiques. Selon un cas avantageux, un tel médicament est notamment destiné au traitement des cancers gastriques et du colon.

En vue de son administration à l'homme ou l'animal, une telle composition comprend un support acceptable du point de vue pharmaceutique.

L'invention concerne donc aussi l'utilisation d'une séquence nucléique, d'un vecteur, ou d'une cellule hôte telle que définie précédemment pour la préparation d'un médicament destiné au traitement à but préventif, curatif ou vaccinal du corps humain ou animal, et plus particulièrement destiné à un traitement par thérapie génique, des cancers gastriques et du colon.

Le médicament peut être administré sous forme injectable, notamment par voie intramusculaire, intratrachéale, intratumorale, intragastrisque, intrapéritonéale, épidermique, intraveineuse, intraartérielle, par inhalation, par instillation, par aérosolisation, par voie topique ou par voie orale.

Un autre objet de l'invention concerne l'utilisation d'un peptide de l'invention pour la préparation d'une composition diagnostique, prophylactique ou thérapeutique, notamment une composition vaccinale dont l'administration à un patient est capable d'induire une réponse immunitaire caractérisée par une réponse CTL. L'invention concerne donc aussi toute composition diagnostique, prophylactique ou thérapeutique comprenant au moins un peptide de l'invention.

A titre d'exemples d'utilisations diagnostiques d'un peptide de l'invention, il est possible de citer les procédés consistant à détecter, et éventuellement quantifier :
- un anticorps dirigé contre ledit peptide, en mettant en contact un échantillon biologique susceptible de contenir ledit anticorps avec ledit peptide, puis en détectant la formation d'un complexe immun entre l'anticorps et le peptide par des techniques connues de l'homme du métier,
- les lymphocytes T cytotoxiques selon la technique dite ELISPOT décrite par Scheibenbogen et al, 1997, Clinical Cancer Research, 3, 221-226, dont le contenu fait partie intégrante de la présente demande. Une telle détermination est particulièrement avantageuse lorsque l'on souhaite évaluer l'efficacité d'une approche vaccinale mise en oeuvre chez un patient donné ou pour diagnostiquer un état pathologique potentiel chez un patient en cherchant à mettre en évidence une réponse immune naturellement développée par ledit patient contre l'antigène TGF beta RII muté.

Les caractéristiques et avantages de l'invention sont illustrés dans les exemples suivants, ainsi qu'à l'appui des figures.

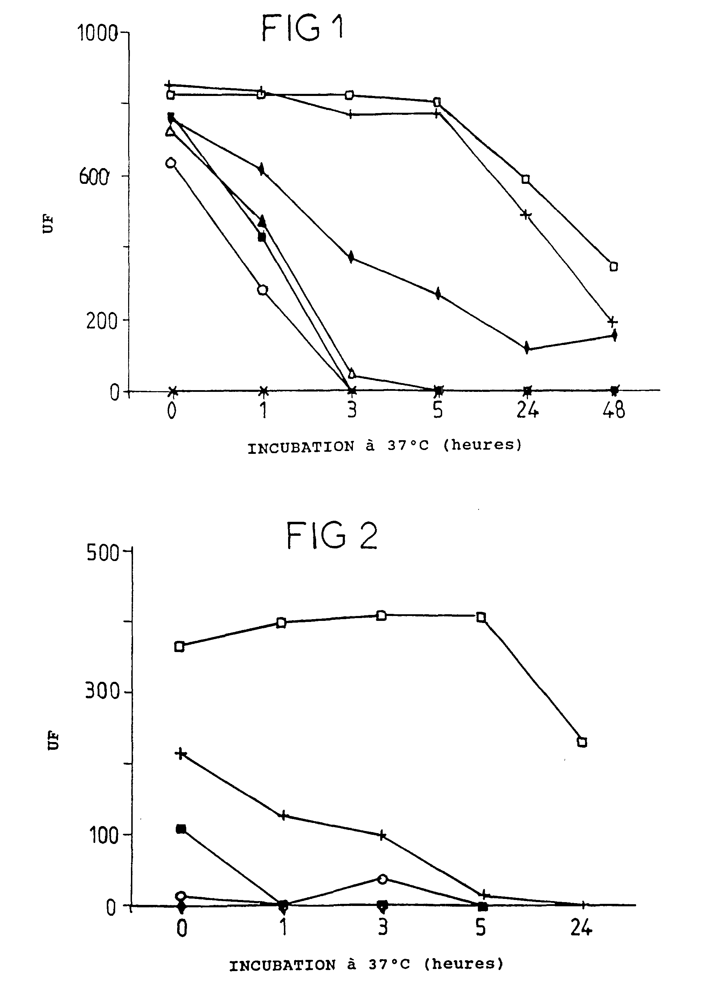

La figure 1 représente l'effet de la durée d'incubation pour différents peptides sur leur association avec les molécules HLA-A2, la détection correspondant à une lecture à 340/460 nm à l'aide d'un cytofluorimètre et étant exprimée en unité de fluorescence U.F ; la légende de la figure est la suivante :
- □: peptide de contrôle positif
- X: peptide de contrôle négatif
- ◆: peptide de SEQ ID NO: 4
- Δ: peptide de SEQ ID NO: 2
- +: peptide de SEQ ID NO: 5
- ○: peptide de SEQ ID NO: 6
- ■: peptide de SEQ ID NO: 3

La figure 2 représente l'effet de la durée d'incubation pour différents peptides sur leur association avec les molécules HLA-A3, la détection correspondant à une lecture à 340/460 nm à l'aide d'un cytofluorimètre et étant exprimée en unité de fluorescence U.F ; la légende de la figure est la suivante :
- D: peptide de contrôle positif
- ◆: peptide de contrôle négatif
- ■: peptide de SEQ ID NO: 7
- ○: peptide de SEQ ID NO: 8
- +: peptide de SEQ ID NO: 9

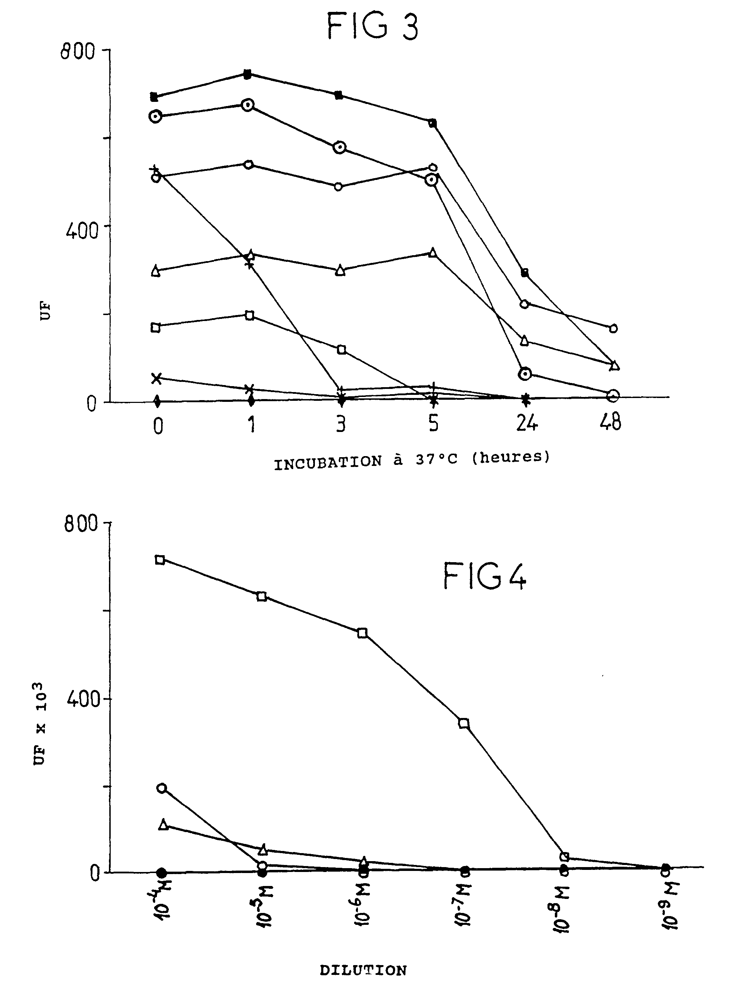

La figure 3 représente l'effet de la durée d'incubation pour-différents peptides sur leur association avec les molécules HLA-B8, la détection correspondant à une lecture à 340/460 nm à l'aide d'un cytofluorimètre et étant exprimée en unité de fluorescence U.F ; la légende de la figure est la suivante :
- ■: peptide de contrôle positif
- ◆: peptide de contrôle négatif
- ○: peptide de SEQ ID NO: 10
- △: peptide de SEQ ID NO: 17
- ⊙: peptide de SEQ ID NO: 11
- X: peptide de SEQ ID NO: 12
- +: peptide de SEQ ID NO: 13
- □: peptide de SEQ ID NO: 15

La figure 4 représente l'effet de la dilution de différents peptides sur leur association avec les molécules HLA-B7, la détection correspondant à une lecture à 340/460 nm à l'aide d'un cytofluorimètre et étant exprimée en unité de fluorescence U.F ; la légende de la figure est la suivante :
- □: peptide de contrôle positif
- ●: peptide de contrôle négatif
- △: peptide de SEQ ID NO: 20
- ○: peptide de SEQ ID NO: 13

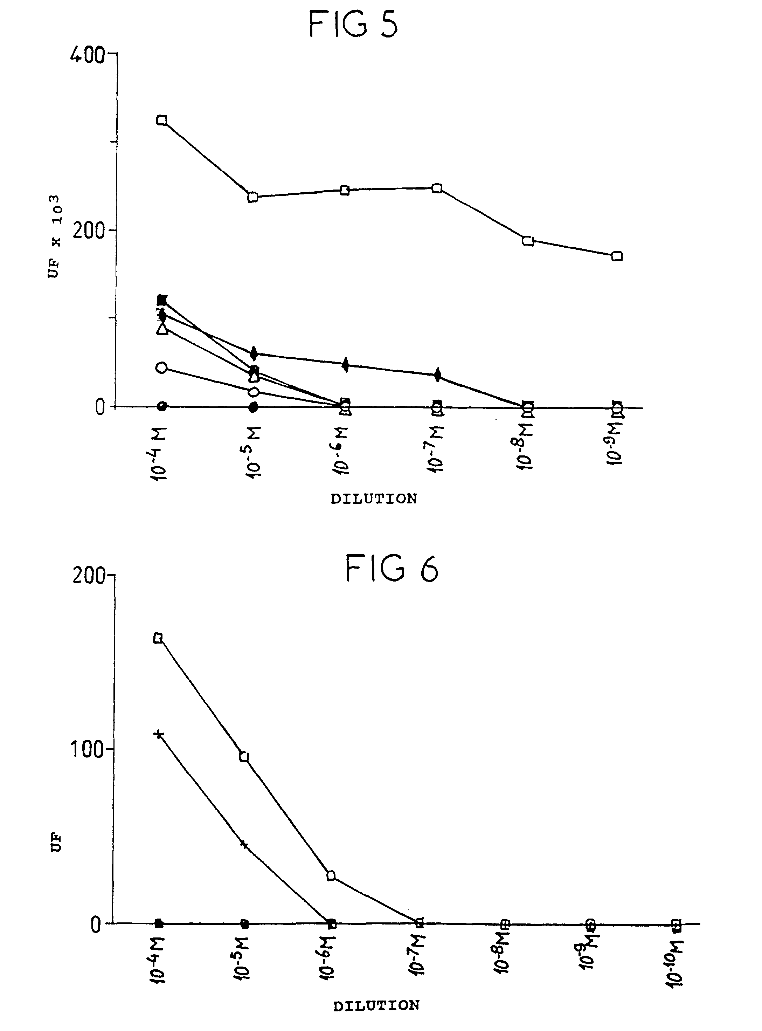

La figure 5 représente l'effet de la dilution de différents peptides sur leur association avec les molécules HLA-B35, la détection correspondant à une lecture à 340/460 nm à l'aide d'un cytofluorimètre et étant exprimée en unité de fluorescence U.F ; la légende de la figure est la suivante :
- □: peptide de contrôle positif
- ●: peptide de contrôle négatif
- ■: peptide de SEQ ID NO: 18
- Δ: peptide de SEQ ID NO: 19
- ◆: peptide de SEQ ID NO: 20
- ○: peptide de SEQ ID NO: 13

La figure 6 représente l'effet de la dilution du peptide de SEQ ID NO: 21 sur son association avec les molécules HLA-B27, la détection correspondant à une lecture à 340/460 nm à l'aide d'un cytofluorimètre et étant exprimée en unité de fluorescence U.F; la légende de la figure est la suivante : peptide de SEQ ID NO: 21 (N 31), peptide de contrôle positif et peptide de contrôle négatif.
- □: peptide de contrôle positif
- ■: peptide de contrôle négatif
- +: peptide de SEQ ID NO: 21

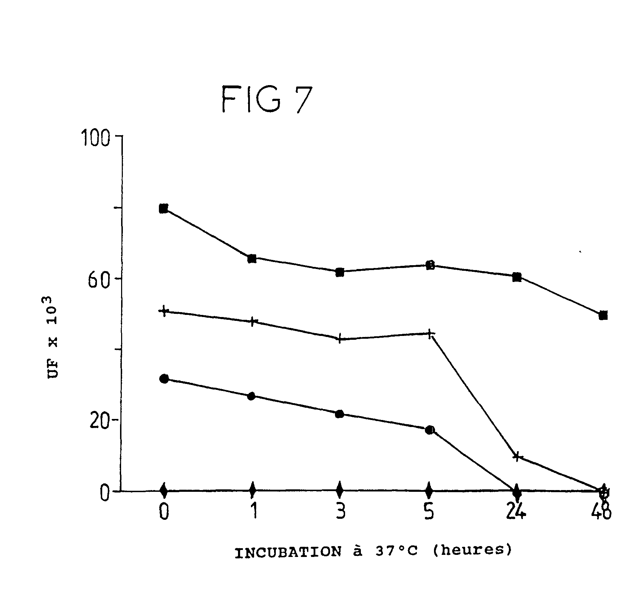

La figure 7 représente l'effet de la durée d'incubation du peptide de SEQ ID NO: 21, sur son association avec les molécules HLA-B27, la détection correspondant à une lecture à 340/460 nm à l'aide d'un cytofluorimètre et étant exprimée en unité de fluorescence U.F; la légende de la figure est la suivante :
■ peptide de contrôle positif
◆ peptide de contrôle négatif
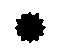 peptide de SEQ ID NO: 21

### Exemple 1 : Détection de l'association entre les peptides et les molécules d'histocompatibilité

### 1) Matériel

### - Sources de molécules d'histocompatibilité

Elles sont actuellement de deux types principaux : les cellules mutantes et les molécules d'histocompatibilité purifiées.

La cellule mutante utilisée est la cellule humaine T2 qui est un variant de la lignée Tl produite par fusion du lymphome T CEM et du lymphome B 721.174 (Salter and Cresswell, EMBO J., 1986, 5(5) 943-949). Cette cellule qui est dépourvue de transporteurs de peptides contient des chaînes lourdes de molécules de classe I libres de peptides qui vont pouvoir accepter des peptides exogènes.

Des molécules d'histocompatibilité de classe I purifiées par chromatographie d'affinité à partir de lignées de cellules B humaines transformées par l'EBV peuvent être également utilisées. Dans ce cas les peptides endogènes doivent être éliminés par un traitement avec de l'urée 1,5 M et de la soude 12,5 mM (pH 11,7) pendant 1 h à 4°C, suivi de leur élimination par une colonne de désalage (PDLO, Pharmacia). Les molécules d'histocompatibilité sont immédiatement remises en présence des peptides à tester dans un tampon PBS avec 0,05 % Tween 20, 2 mM EDTA, 0,1 % NP40 et 6 mM CHAPS, en présence de 2 *µ*g/ml B2m pour faciliter la réassociation (Gnjatic et al., Eur. J. Immunol., 1995, 25, 1638-1642).

### - Peptides

Les peptides testés ont en général 8 à 10 résidus, parfois 11 ou 12. Ils ont été synthétisés par Néosystem (Strasbourg), ou par CHIRON mimotopes (Victoria, Australie). Ils sont utilisés à des concentrations variant de 100 *µ*M à 0,1 nM.

### 2) Protocole de l'assemblage (Connan et al., Eur. J. Immunol., 1994, 24:777 ; Couillin et al., Eur. J. Immunol., 1995, 25, 728-732)

Des aliquotes de 8 x 10⁵ cellules dans un volume de 64 *µ*l, répartis dans des tubes microfuge Eppendorf, sont mis en présence d'un tampon de lyse contenant 10 mM PBS, pH 7,5, 1 % NP40, des inhibiteurs de protéases (1 mM PMSF, 100 *µ*M iodoacétamide, 2 *µ*g/ml aprotinine, 10 *µ*M leupeptine, 10 *µ*M pepstatine et 10 *µ*g/ml inhibiteur de trypsine). La lyse se fait en présence des peptides à tester pendant 30 min ou 1 h à 37°C. Après élimination du matériel non solubilisé par une centrifugation à 15 000 tours/min à 4°C, le surnageant est additionné de 140 *µ*l de PBS contenant 0,05 % de Tween 20, 3 mM d'azide de sodium, 1 mM PMSF et 10 mg/ml d'albumine bovine. Chaque échantillon est incubé pendant 20 h à 4°C dans 2 puits d'une plaque à microtitration de type Nunc, Maxisorb, préalablement recouverts d'un anticorps monoclonal (10 *µ*g/ml en PBS) qui reconnaît les molécules d'histocompatibilité ayant une(des) conformation(s) conforme(s) pour la présentation des peptides et semblable(s) à celle(s) présente(s) à la surface des cellules. La plaque recouverte d'anticorps est préalablement saturée par de l'albumine bovine à 10 mg/ml dans du PBS-Tween avant la mise de l'échantillon. Le second anticorps qui permet la détection de l'assemblage des molécules d'histocompatibilité est dirigé contre la B2m. Il est couplé soit à la biotine (NHS-LC biotin, Perce) soit à la phosphatase alcaline (P-552, Sigma) et est incubé à 2 *µ*g/ml pendant une heure à 37°C. Dans le cas de l'emploi de la biotine, une incubation de 45 minutes à 20-25°C avec de l'extravidine couplée à la phosphatase alcaline (E-2636, Sigma) est réalisée. L'activité de la phosphatase alcaline est mesurée en utilisant comme substrat le 4-méthyl-umbelliféryl-phosphate (M-8883, Sigma) à 100 *µ*M dans de la diéthanolamine 50 mM, pH 9,5 avec du MgCl₂ 1 mM. La lecture est faite à 340/460 nm à l'aide d'un cytofluorimètre.

### 3) Stabilité des complexes HLA/peptides

La stabilité des complexes précités a été étudiée car elle conditionne la bonne présentation de l'antigène et l'induction de la réponse T.

A cet effet, on a utilisé soit du HLA purifié, soit le lysat de la cellule T2.

Avec le HLA purifié, on a éliminé les peptides endogènes [comme décrit en 2)] puis on l'a mis en présence du peptide à tester en tube Eppendorf à 37°C, pendant des temps variables de quelques minutes à plusieurs jours. La phase suivante d'incubation sur plaque de 96 puits [comme décrit en 2)] avec l'anticorps anti-HLA se fait pendant une heure à 37°C. La révélation est effectuée de manière classique.

Avec le lysat de la cellule T2, toutes les incubations sont également faites à 37°C, après ajout de tous les inhibiteurs de protéases.

Les exemples ci-après ont pour but d'illustrer les applications potentielles des peptides de l'invention.

### Exemple 2 : Préparation d'anticorps

### - Immunisation des lapins et des souris

L'immunogène suivant a été préparé : le peptide de SEQ ID NO: 43 couplé à de l'hémocyanine de Lymphet Keyhole, en abrégé peptide-KLH, comme support pour son utilisation en immunisation, et ledit peptide couplé à de l'albumine de sérum humain, en abrégé peptide-HSA, destiné aux tests ELISA.

Les lapins ont été soumis à une injection de peptide-KLH en utilisant de l'adjuvant complet de Freund (CFA) puis deux injections du même peptide en utilisant de l'adjuvant incomplet de Freund (IFA). Les souris ont été soumises aux mêmes injections en suivant le même protocole, à l'exception de quelques souris qui ont été immunisées par injection de peptide-HSA.

Les sérums et les surnageants de culture d'hybridome issus des animaux immunisés par le peptide (SEQ ID NO: 43)-HSA ont été analysés par test ELISA, en utilisant les peptides-KLH, et inversement.

### - Obtention d'anticorps monoclonaux

On a recherché et analysé chez les souris immunisées comme décrit ci-dessus, les anticorps dirigés contre le peptide SEQ ID NO: 43. Les souris immunisées par les peptides-HSA possèdent le titre en anticorps le plus élevé. Les cellules spléniques de ces souris ont par conséquent été récupérées et fusionnées avec des cellules de myélome. Les surnageants de culture d'hybridomes ont été analysés par test ELISA du point de vue de leur réactivité avec les peptides de l'invention et des cellules infectées par le virus recombinant de la vaccine WTG-8048 (cf ci-après).

### Exemple 3 : Matériels et méthodes pour d'autres utilisations des peptides de l'invention

### 1) Obtention du virus recombinant de la vaccine (VVTG-8048)

La séquence complète décalée (décalage du cadre de lecture de -1) du récepteur RII du TGFβ, dénommée RIIf, a été clonée dans le plasmide pTG186 en utilisant la thymidine kinase (TK) pour le site d'insertion et le promoteur p7.5. Ce produit d'assemblage a été transféré dans le virus de la vaccine pour obtenir le virus recombinant dénommé VVTG-8048.

La même séquence complète décalée précitée a été clonée dans le plasmide pTG186 au site d'insertion del-3 et promoteur p7.5 pour obtenir un produit d'assemblage dans le système MVA. Ce produit porte à la fois la séquence précitée et la séquence de l'IL-2, qui ont le même site d'insertion, mais la séquence RIIf est régie par le promoteur p7.5 et la séquence de IL-2 est régie par le promoteur H5R.

### 2) Western blots

Des cultures de cellules de BHK ont été infectées par VVTG-8048 pendant 24 heures. Les cellules ont ensuite été lysées par sonication. Les surnageants de culture ainsi que les culots de sonication ont été analysés en Western Blots.

Des extraits de E. Coli 8051 et 8067 ont été enrichis en RIIf par passage sur résine chélatée à du nickel (fournie par Qiagen) selon le protocole qui sera décrit plus loin.

Les deux types d'extraits ont été testés en Western Blot dans des conditions dénaturantes (dans du dodécylsulfate de sodium, SDS). L'expression de la protéine RII a été révélée par un antisérum polyclonal de lapin, disponible dans le commerce (Santa Cruz, SC1700) et dirigé contre la forme native de RII. Cet antisérum reconnaît en outre les épitopes de la molécule mutée.

### 3) Protéine recombinante

La protéine recombinante correspondant à la séquence RIIf a été exprimée dans E. coli comme protéine recombinante avec soit poly-His, soit poly-His + épitope 5F3 destiné à l'immunopurification. La fraction protéique insoluble est solubilisée dans de l'urée 8 M. L'enrichissement du produit a été effectué sur résine chélatée à du nichel (Qiagen). La colonne a été lavée avec des concentrations décroissantes d'urée. L'élution a été faite avec de l'imidazole en l'absence d'urée.

### 4) Expression chez le mammifère

La séquence RIIf a été entièrement clonée dans pCDNA-3 pour une transfection dans des cellules de mammifères. Les cellules Balb/c 3T3, RMA et P815 ont été transfectée par électroporation et sélectionnées dans 1 mg/ml G418. Des cellules Balb/c 3T3 (H-2d) ont été transfectées par CaPO₄. Les cellules P815-HT (H-2d aussi) ont été transfectées par CaPO₄. Les cellules RMA (H-2d) ont été transfectées par la lipofectine et par CaPO₄.

Les clones résistants au G418 ont été testés pour l'expression de l'insert RIIF par PCR. Plusieurs clones sont capables d'exprimer l'insert RIIF.

### Exemple 4 : Mise en évidence in vivo du pouvoir immunogène du peptide de SEQ ID NO :5

On a également étudié *in vivo* le pouvoir immunogène des peptides de l'invention. Ce pouvoir immunogène est évalué par rapport à la capacité qu'ont ces peptides à induire des effecteurs T cytolytiques chez la souris transgénique A2/R^{b} exprimant le HLA/A2 (Correale et al, J. Immunol. 1998 Sep 15 ; 161(6) :3186-94). Le peptide de SEQ ID NO : 5 (RLSSCVPVA) a été choisi pour illustrer cette étude.

Dans un premier temps, les souris sont co-injectées avec deux épitopes : le peptide SEQ ID NO : 5 qui se fixe à la molécule de HLA-A2, et un peptide à effet « helper », le peptide HBVc 128-140, dérivé de la protéine « core » du virus de l'hépatite B, qui est reconnu par les lymphocytes TCD4+ des souris. Une injection sous cutanée est réalisée à la base de la queue à trois reprises (J0), avec 200 *µ*l d'une émulsion en adjuvant de Freund incomplet (IFA) contenant 50 *µ*g du peptide de SEQ ID NO : 5 (2 mg/ml dans NaCl) et 140 *µ*g du peptide HBVc 128-140 (2 mg/ml dans NaCl).

L'expérience a été effectuée sur un lot de 6 souris mâles comprenant :
- 2 souris immunisées avec 200 *µ*l IFA + NaCl + HBVc 128-140 (souris p0),
- 2 souris immunisées avec 200 *µ*l IFA + NaCl + HBVc 128-140 + peptide de SEQ ID NO : 5 (souris p5) et,
- 2 souris immunisées avec 200 *µ*l IFA + peptide HBVc 128-140 + peptide MART 27-35 comme épitope T (Cornier et al, 1997, Cancer J Sci Am ; 3(1) :37-44).

Pour mettre en évidence la présence d'effecteurs T cytolytiques, on procède ensuite à une stimulation supplémentaire *in vivo* (à J11). Cette stimulation est réalisée avec 3.10⁶ splénocytes / ml et 10⁶ blastes induits par LPS et irradiés. Les blastes ont été préparés à J8 à partir de splénocytes de souris non traitées en utilisant 1,5. 10⁶ cellules/ml de RPMI - 10% SVF, 25 *µ*g/ml LPS (1 mg/ml en NaCl), 7 *µ*g/ml de sulfate de dextran (7 mg/ml dans l'eau). A J11, les blastes sont irradiés (4000 rads), lavés et mis en suspension à une concentration de 40.10⁶ cellules / ml avec le peptide de SEQ ID NO : 5 (100 *µ*g/ml) pendant une heure à 37°C. Après lavage, la concentration des blastes est ajustée à 10⁷ cellules / ml puis les blastes sont mélangés à des splénocytes des souris immunisées. La recherche d'effecteurs T cytolytiques est réalisée en effectuant le test CRT (Chromium release Test, Mariani et al, 1994, J Immunol Methods. 24 ;172(2) :173-8).

| | % de lyse |
|---|---|
| -souris p0 + 0 | 0 |
| -souris p0 + p5 | 0 |
| -souris pMART + 0 | 0 |
| -souris pMART + pMART | 0 |
| -souris p5 + 0 | 0 |
| -souris p5 + P5 | 37 |

Les résultats du test CRT mettent en évidence la capacité du peptide de SEQ ID NO : 5 à induire des effecteurs T cytolytiques.

## Revendications

1. Peptide comprenant ou consistant en une première séquence peptidique d'au plus 20 acides aminés consécutifs définis dans une seconde séquence peptidique codée par SEQ ID NO: 42 et différente de SEQ ID NO: 1, ledit peptide étant capable de se fixer sur au moins une glycoprotéine du complexe majeur d'histocompatibilité de classe I (CMH I).

2. Peptide selon la revendication 1, caractérisé en ce que la première séquence est choisie parmi SEQ ID NOS: 2 à 21.

3. Peptide selon la revendication 2, caractérisé en ce que la première séquence est choisie parmi SEQ ID NOS: 2 à 6, ledit peptide étant capable de se fixer sur les glycoprotéines HLA-A2 du CMH-I.

4. Peptide selon la revendication 2, caractérisé en ce que la première séquence est choisie parmi SEQ ID NOS: 7 à 9, ledit peptide étant capable de se fixer sur les glycoprotéines HLA-A3 du CMH-I.

5. Peptide selon la revendication 2, caractérisé en ce que la première séquence est choisie parmi SEQ ID NOS: 7 à 9, ledit peptide étant capable de se fixer sur les glycoprotéines HLA-A11 du CMH-I.

6. Peptide selon la revendication 2, caractérisé en ce que la première séquence est choisie parmi SEQ ID NOS: 10 à 17, ledit peptide étant capable de se fixer sur les glycoprotéines HLA-B8 du CMH-I.

7. Peptide selon la revendication 2, caractérisé en ce que la première séquence est choisie parmi SEQ ID NOS: 18 et 19, ledit peptide étant capable de se fixer sur les glycoprotéines HLA-B7 du CMH-I.

8. Peptide selon la revendication 2, caractérisé en ce que la première séquence est SEQ ID NO: 20, ledit peptide étant capable de se fixer sur les glycoprotéines HLA-B35 du CMH-I.

9. Peptide selon la revendication 2, caractérisé en ce que la première séquence est SEQ ID NO: 21, ledit peptide étant capable de se fixer sur les glycoprotéines HLA-B27 du CMH-I.

10. Peptide selon la revendication 2, caractérisé en ce que la première séquence est SEQ ID NO: 2, ledit peptide étant capable de se fixer sur les glycoprotéines HLA-B62 du CMH-I.

11. Séquence nucléique codant pour un peptide selon l'une quelconque des revendications 1 à 10.

12. Séquence selon la revendication 11, caractérisée en ce qu'elle est choisie parmi SEQ ID NOS: 22 à 41, et leurs séquences complémentaires.

13. Vecteur de clonage ou d'expression comportant au moins une séquence nucléique selon la revendication 11 ou 12, et des éléments nécessaires à son expression dans une cellule hôte.

14. Vecteur selon la revendication 13, caractérisé en ce qu'il comporte au moins deux séquences nucléiques différentes respectivement choisies :
parmi les séquences codant pour un peptide de la revendication 3 et les séquences codant pour un peptide de l'une quelconque des revendications 4 à 10,
parmi les séquences codant pour un peptide de la revendication 4 et les séquences codant pour un peptide de l'une quelconque des revendications 3 et 5 à 10,
parmi les séquences codant pour un peptide de la revendication 5 et les séquences codant pour un peptide de l'une quelconque des revendications 3, 4 et 6 à 10,
parmi les séquences codant pour un peptide de la revendication 6 et les séquences codant pour un peptide de l'une quelconque des revendications 3 à 5 et 7 à 10,
parmi les séquences codant pour un peptide de la revendication 7 et les séquences codant pour un peptide de l'une quelconque des revendications 3 à 6 et 8 à 10,
parmi les séquences codant pour un peptide de la revendication 8 et les séquences codant pour un peptide de l'une quelconque des revendications 3 à 7, 9 et 10,
parmi les séquences codant pour un peptide de la revendication 9 et les séquences codant pour un peptide de l'une quelconque des revendications 3 à 8 et 10, et
parmi les séquences codant pour un peptide de la revendication 10 et les séquences codant pour un peptide de l'une quelconque des revendications 3 à 9.

15. Vecteur selon la revendication 13 ou 14, caractérisé en ce qu'il est choisi parmi les vecteurs viraux à réplication autonome ou permettant l'intégration chromosomique et parmi les vecteurs non viraux.

16. Vecteur selon la revendication 15, caractérisé en ce qu'il s'agit d'un vecteur viral réalisé sur la base d'un adénovirus, d'un rétrovirus, d'un poxvirus, du virus de la vaccine ou d'un virus herpétique.

17. Vecteur selon la revendication 15, caractérisé en ce qu'il s'agit d'un vecteur non viral consistant en un plasmide éventuellement associé à au moins un composé capable de faciliter l'introduction dudit plasmide dans des cellules à transfecter.

18. Vecteur selon la revendication 17, caractérisé en ce que ledit composé est choisi parmi les lipides cationiques et les polymères cationiques.

19. Cellule hôte comprenant au moins une séquence nucléique selon la revendication 11 ou 12, ou au moins un vecteur selon l'une quelconque des revendications 13 à 18.

20. Composition pharmaceutique comprenant à titre de principe actif au moins une séquence nucléique selon la revendication 11 ou 12, un vecteur selon l'une quelconque des revendications 13 à 18, ou une cellule hôte selon la revendication 19.

21. Composition selon la revendication 20, caractérisée en ce qu'elle est destinée au traitement des cancers gastriques ou du colon.

22. Composition selon la revendication 20 ou 21, caractérisée en ce qu'elle comprend un support acceptable du point de vue pharmaceutique permettant son administration à l'homme ou l'animal.

23. Utilisation d'une séquence nucléique selon la revendication 11 ou 12, d'un vecteur selon l'une quelconque des revendications 13 à 18, ou d'une cellule hôte selon la revendication 19, pour la préparation d'un médicament destiné au traitement à but préventif, curatif ou vaccinal du corps humain ou animal.

24. Utilisation selon la revendication 23, caractérisée en ce que le médicament est destiné à un traitement par thérapie génique.

25. Utilisation selon la revendication 23, caractérisée en ce que le médicament est destiné à traiter des cancers gastriques ou du colon.

26. Utilisation selon la revendication 23, caractérisée en ce que le médicament est destiné à être administré sous forme injectable, notamment par voie intramusculaire, intratrachéale, intratumorale, intragastrisque, intrapéritonéale, épidermique, intraveineuse, intraartérielle, par inhalation, par instillation, par aérosolisation, par voie topique ou par voie orale.

27. Composition diagnostique, prophylactique ou thérapeutique comprenant au moins un peptide selon l'une quelconque des revendications 1 à 10.

28. Utilisation d'un peptide selon l'une quelconque des revendications 1 à 10, pour préparer une composition diagnostique, prophylactique ou thérapeutique, destinée à détecter, prévenir ou traiter des cancers gastriques ou du colon.

## Claims

1. Peptide comprising or consisting of a first peptide sequence of at most 20 consecutive amino acids defined in a second peptide sequence encoded by SEQ ID NO: 42 and different from SEQ ID NO: 1, said peptide being capable of binding to at least one major histocompatibility complex class I (MHC I) glycoprotein.

2. Peptide according to Claim 1, characterized in that the first sequence is chosen from SEQ ID NOS: 2 to 21.

3. Peptide according to Claim 2, characterized in that the first sequence is chosen from SEQ ID NOS: 2 to 6, said peptide being capable of binding to the MHC I HLA-A2 glycoproteins.

4. Peptide according to Claim 2, characterized in that the first sequence is chosen from SEQ ID NOS: 7 to 9, said peptide being capable of binding to the MHC I HLA-A3 glycoproteins.

5. Peptide according to Claim 2, characterized in that the first sequence is chosen from SEQ ID NOS: 7 to 9, said peptide being capable of binding to the MHC I HLA-A11 glycoproteins.

6. Peptide according to Claim 2, characterized in that the first sequence is chosen from SEQ ID NOS: 10 to 17, said peptide being capable of binding to the MHC I HLA-B8 glycoproteins.

7. Peptide according to Claim 2, characterized in that the first sequence is chosen from SEQ ID NOS: 18 and 19, said peptide being capable of binding to the MHC I HLA-B7 glycoproteins.

8. Peptide according to Claim 2, characterized in that the first sequence is SEQ ID NO: 20, said peptide being capable of binding to the MHC I HLA-B35 glycoproteins.

9. Peptide according to Claim 2, characterized in that the first sequence is SEQ ID NO: 21, said peptide being capable of binding to the MHC I HLA-B27 glycoproteins.

10. Peptide according to Claim 2, characterized in that the first sequence is SEQ ID NO: 2, said peptide being capable of binding to the MHC I HLA-B62 glycoproteins.

11. Nucleic sequence encoding a peptide according to any one of Claims 1 to 10.

12. Sequence according to Claim 11, characterized in that it is chosen from SEQ ID NOS: 22 to 41, and their complementary sequences.

13. Cloning or expression vector comprising at least one nucleic sequence according to Claim 11 or 12, and elements necessary for its expression in a host cell.

14. Vector according to Claim 13, characterized in that it comprises at least two different nucleic sequences chosen respectively:
from the sequences encoding a peptide of Claim 3 and the sequences encoding a peptide of any one of Claims 4 to 10,
from the sequences encoding a peptide of Claim 4 and the sequences encoding a peptide of any one of Claims 3 and 5 to 10,
from the sequences encoding a peptide of Claim 5 and the sequences encoding a peptide of any one of Claims 3, 4 and 6 to 10,
from the sequences encoding a peptide of Claim 6 and the sequences encoding a peptide of any one of Claims 3 to 5 and 7 to 10,
from the sequences encoding a peptide of Claim 7 and the sequences encoding a peptide of any one of Claims 3 to 6 and 8 to 10,
from the sequences encoding a peptide of Claim 8 and the sequences encoding a peptide of any one of Claims 3 to 7, 9 and 10,
from the sequences encoding a peptide of Claim 9 and the sequences encoding a peptide of any one of Claims 3 to 8 and 10, and
from the sequences encoding a peptide of Claim 10 and the sequences encoding a peptide of any one of Claims 3 to 9.

15. Vector according to Claim 13 or 14, characterized in that it is chosen from the autonomously replicating viral vectors or the viral vectors allowing chromosomal integration and from the nonviral vectors.

16. Vector according to Claim 15, characterized in that it is a viral vector produced based on an adenovirus, a retrovirus, a poxvirus, the vaccinia virus or a herpesvirus.

17. Vector according to Claim 15, characterized in that it is a nonviral vector consisting of a plasmid optionally combined with at least one compound capable of facilitating the introduction of said plasmid into cells to be transfected.

18. Vector according to Claim 17, characterized in that said compound is chosen from the cationic lipids and the cationic polymers.

19. Host cell comprising at least one nucleic sequence according to Claim 11 or 12, or at least one vector according to any one of Claims 13 to 18.

20. Pharmaceutical composition comprising, as active ingredient, at least one nucleic sequence according to Claim 11 or 12, a vector according to any one of Claims 13 to 18, or a host cell according to Claim 19.

21. Composition according to Claim 20, characterized in that it is intended for the treatment of gastric or colon cancers.

22. Composition according to Claim 20 or 21, characterized in that it comprises a pharmaceutically acceptable carrier allowing its administration to humans or animals.

23. Use of a nucleic sequence according to Claim 11 or 12, of a vector according to any one of Claims 13 to 18, or of a host cell according to Claim 19, for the preparation of a medicament intended for the preventive, curative or vaccinal treatment of the human or animal body.

24. Use according to Claim 23, characterized in that the medicament is intended for a gene therapy treatment.

25. Use according to Claim 23, characterized in that the medicament is intended for treating gastric or colon cancers.

26. Use according to Claim 23, characterized in that the medicament is intended to be administered in injectable form, in particular by the intramuscular, intratracheal, intratumoral, intragastric, intraperitoneal, epidermal, intravenous or intraarterial route, by inhalation, by instillation, by aerosolization, by the topical route or by the oral route.

27. Diagnostic, prophylactic or therapeutic composition comprising at least one peptide according to any one of Claims 1 to 10.

28. Use of a peptide according to any one of Claims 1 to 10, for preparing a diagnostic, prophylactic or therapeutic composition intended for detecting, preventing or treating gastric or colon cancers.

## Patentansprüche

1. Peptid, das eine erste Peptidsequenz von höchtens 20 aufeinanderfolgenden Aminosäuren aufweist oder daraus besteht, wobei die 20 aufeinanderfolgenden Aminosäuren in einer zweiten Peptidsequenz definiert sind, die durch SEQ ID NO: 42 kodiert und verschieden von SEQ ID NO: 1 ist, und das Peptid in der Lage ist, an zumindest ein Glykoprotein des Haupt-Histokompatibilitäts-Komplex Klasse I (MHC I) zu binden.

2. Peptid nach Anspruch 1, dadurch gekennzeichnet, daß die erste Sequenz ausgewählt ist aus SEQ ID Nr. 2 bis 21.

3. Peptid nach Anspruch 2, dadurch gekennzeichnet, daß die erste Sequenz ausgewählt ist aus SEQ ID Nr. 2 bis 6, wobei das Peptid in der Lage ist, an die Glykoproteine HLA-A2 des MHC-I zu binden.

4. Peptid nach Anspruch 2, dadurch gekennzeichnet, daß die erste Sequenz ausgewählt ist aus SEQ ID Nr. 7 bis 9, wobei das Peptid in der Lage ist, an die Glykoproteine HLA-A3 des MHC-I zu binden.

5. Peptid nach Anspruch 2, dadurch gekennzeichnet, daß die erste Sequenz ausgewählt ist aus SEQ ID Nr. 7 bis 9, wobei das Peptid in der Lage ist, an die Glykoproteine HLA-All des MHC-I zu binden.

6. Peptid nach Anspruch 2, dadurch gekennzeichnet, daß die erste Sequenz ausgewählt ist aus SEQ ID Nr. 10 bis 17, wobei das Peptid in der Lage ist, an die Glykoproteine HLA-B8 des MHC-I zu binden.

7. Peptid nach Anspruch 2, dadurch gekennzeichnet, daß die erste Sequenz ausgewählt ist aus SEQ ID Nr. 18 und 19, wobei das Peptid in der Lage ist, an die Glykoproteine HLA-B7 des MHC-I zu binden.

8. Peptid nach Anspruch 2, dadurch gekennzeichnet, daß die erste Sequenz SEQ ID Nr. 20 ist, wobei das Peptid in der Lage ist, an die Glykoproteine HLA-B35 des MHC-I zu binden.

9. Peptid nach Anspruch 2, dadurch gekennzeichnet, daß die erste Sequenz SEQ ID Nr. 21 ist, wobei das Peptid in der Lage ist, an die Glykoproteine HLA-B27 des MHC-I zu binden.

10. Peptid nach Anspruch 2, dadurch gekennzeichnet, daß die erste Sequenz SEQ ID Nr. 2 ist, wobei das Peptid in der Lage ist, an die Glykoproteine HLA-B62 des MHC-I zu binden.

11. Nukleinsequenz, die für ein Peptid nach einem der Ansprüche 1 bis 10 kodiert.

12. Sequenz nach Anspruch 11, dadurch gekennzeichnet, daß sie ausgewählt ist aus SEQ ID Nr. 22 bis 41 und deren komplementären Sequenzen.

13. Klonierungs- oder Expressionsvektor, der zumindest eine Nukleinsequenz nach Anspruch 11 oder 12 sowie Elemente enthält, die für ihre Expression in einer Wirtszelle notwendig sind.

14. Vektor nach Anspruch 13, dadurch gekennzeichnet, daß er zumindest zwei unterschiedliche Nukleinsequenzen enthält, jeweils ausgewählt:
aus den Sequenzen, die für ein Peptid nach Anspruch 3 kodieren, und den Sequenzen, die für ein Peptid nach einem der Ansprüche 4 bis 10 kodieren,
aus den Sequenzen, die für ein Peptid nach Anspruch 4 kodieren, und den Sequenzen, die für ein Peptid nach einem der Ansprüche 3 und 5 bis 10 kodieren,
aus den Sequenzen, die für ein Peptid nach Anspruch 5 kodieren, und den Sequenzen, die für ein Peptid nach einem der Ansprüche 3, 4 und 6 bis 10 kodieren,
aus den Sequenzen, die für ein Peptid nach Anspruch 6 kodieren, und den Sequenzen, die für ein Peptid nach einem der Ansprüche 3 bis 5 und 7 bis 10 kodieren,
aus den Sequenzen, die für ein Peptid nach Anspruch 7 kodieren, und den Sequenzen, die für ein Peptid nach einem der Ansprüche 3 bis 6 und 8 bis 10 kodieren,
aus den Sequenzen, die für ein Peptid nach Anspruch 8 kodieren, und den Sequenzen, die für ein Peptid nach einem der Ansprüche 3 bis 7, 9 und 10 kodieren,
aus den Sequenzen, die für ein Peptid nach Anspruchs 9 kodieren, und den Sequenzen, die für ein Peptid nach einem der Ansprüche 3 bis 8 und 10 kodieren, und
aus den Sequenzen, die für ein Peptid nach Anspruch 10 kodieren, und den Sequenzen, die für ein Peptid nach einem der Ansprüche 3 bis 9 kodieren.

15. Vektor nach Anspruch 13 oder 14, dadurch gekennzeichnet, daß er ausgewählt ist aus den viralen Vektoren mit autonomer Replikation oder aus den viralen Vektoren, die die chromosomale Integration ermöglichen, und aus den nicht-viralen Vektoren.

16. Vektor nach Anspruch 15, dadurch gekennzeichnet, daß es sich um einen viralen Vektor handelt, der auf der Basis eines Adenovirus, eines Retrovirus, eines Pockenvirus, eines Kuhpockenvirus oder eines Herpesvirus realisiert ist.

17. Vektor nach Anspruch 15, dadurch gekennzeichnet, daß es sich um einen nicht-viralen Vektor handelt, der aus einem Plasmid besteht, das gegebenenfalls mit zumindest einer Zusammensetzung assoziiert ist, die in der Lage ist, das Einbringen des Plasmides in die zu transfizierenden Zellen zu erleichtern.

18. Vektor nach Anspruch 17, dadurch gekennzeichnet, daß die Zusammensetzung ausgewählt ist aus den kationischen Lipiden und den kationischen Polymeren.

19. Wirtszelle, die zumindest eine Nukleinsequenz nach Anspruch 11 oder 12 oder zumindest einen Vektor nach einem der Ansprüche 13 bis 18 enthält.

20. Pharmazeutische Zusammensetzung, die als Wirkstoff zumindest eine Nukleinsequenz nach Anspruch 11 oder 12, einen Vektor nach einem der Ansprüche 13 bis 18, oder eine Wirtszelle nach Anspruch 19 enthält.

21. Zusammensetzung nach Anspruch 20, dadurch gekennzeichnet, daß sie zur Behandlung von Magen- oder Colonkrebs vorgesehen ist.

22. Zusammensetzung nach Anspruch 20 oder 21, dadurch gekennzeichnet, daß sie einen in pharmazeutischer Hinsicht akzeptablen Träger aufweist, der ihre Verabreichung an Mensch oder Tier ermöglicht.

23. Verwendung einer Nukleinsequenz nach Anspruch 11 oder 12, eines Vektors nach einem der Ansprüche 13 bis 18, oder einer Wirtszelle nach Anspruch 19 für die Zubereitung eines Medikamentes, das zur präventiven, heilenden oder vakzinalen Behandlung des menschlichen oder tierischen Körpers vorgesehen ist.

24. Verwendung nach Anspruch 23, dadurch gekennzeichnet, daß das Medikament für eine Behandlung mittels Gentherapie vorgesehen ist.

25. Verwendung nach Anspruch 23, dadurch gekennzeichnet, daß das Medikament zur Behandlung von Magen- oder Colonkrebs vorgesehen ist.

26. Verwendung nach Anspruch 23, dadurch gekennzeichnet, daß das Medikament zur Verabreichung in injizierbarer Form, insbesondere auf intramuskulärem, intratrachealem, intratumoralem, intragastralem, intraperitonealem, epidermalem, intravenösem oder intraarteriellem Wege, durch Inhalation, durch Instillation, durch Aerosolisation, auf topischem Wege oder auf oralem Wege vorgesehen ist.

27. Diagnostische, prophylaktische oder therapeutische Zusammensetzung, die zumindest ein Peptid nach einem der Ansprüche 1 bis 10 enthält.

28. Verwendung eines Peptides nach einem der Ansprüche 1 bis 10, um eine diagnostische, prophylaktische oder therapeutische Zusammensetzung zuzubereiten, die dazu vorgesehen ist, Magen- oder Colonkrebs zu detektieren, vorzubeugen oder zu behandeln.
